# EUROPEAN PATENT APPLICATION

(11) **EP 4 302 772 A1**
(43) Date of publication of application: **10.01.2024**
(21) Application number: 22183390.8
(22) Date of filing: 06.07.2022
(51) Int. Cl.: A61K 36/03, A61P 35/00, A61P 31/00, A61P 37/02, A61K 36/02, A61K 31/737, A61K 8/65

(54) **COMPOSITION FOR ORAL ADMINISTRATION AND SUPPLEMENT COMPRISING SUCH A COMPOSITION**

(71) Applicant: Müller, Jan Allan, 8700 Horsens (DK); Elttør, Eystein E., 700 Klaksvik (FO); Larsen, Lars, 8700 Horsens (DK)
(72) Inventor: Müller, Jan Allan, 8700 Horsens (DK); Elttør, Eystein E., 700 Klaksvik (FO); Larsen, Lars, 8700 Horsens (DK)
(74) Representative: AWA Denmark A/S

(57) **Abstract**

A composition for oral administration, comprising fucoidan from a first source of fucoidan and from a second source of fucoidan, the first source of fucoidan being dried seaweed, and the second source of fucoidan being a fucoidan extract from seaweed. The composition has a weight ratio of the second source to the first source of fucoidan in the range 10 % to 50 % and the first source and second source constitutes at least 60 % by weight of the composition.

## Description

### Field of the invention

The present invention relates to compositions for oral administration comprising fucoidan.

### Background

Fucoidan is a structurally diverse group of fucose-rich sulphated polysaccharides found in brown seaweeds (*Phaeophyceae*), for example in *Laminaria japonica* or *Fucus vesiculosus.* Fucoidans mainly consist of a backbone of α-L-fucopyranose units, but with varying glycosidic links, sulphate positions, sulphation degrees inter alia. Fucoidans have been found to exhibit a number of biological activities which may promote health in animals and humans. Such biological activities include antioxidant, anti-inflammatory, immunoregulatory, antiviral, and anti-tumour effects, see for example Peng, Y. et al, In vitro and in vivo immunomodulatory effects of fucoidan compound agents. Int J Biol Macromol. 2019 Apr 15;127:48-56, and Lin, Y. et al, The anti-cancer effects of fucoidan: a review of both in vivo and in vitro investigations. Cancer Cell Int 20, 154 (2020). A review of the structure and biological activities of fucoidan is found in Ale MT, Mikkelsen JD, Meyer AS, Important determinants for fucoidan bioactivity: a critical review of structure-function relations and extraction methods for fucose-containing sulphated polysaccharides from brown seaweeds, Mar Drugs. 2011;9(10):2106-2130.

Hence, there is an increasing interest in using fucoidan to promote health, and studies indicate that fucoidan intake could improve the immune response to inflammation, viruses and cancer. Achieving these beneficial effects however requires an effective amount of fucoidan being taken up by a user and being made available in the user system. Fucoidan being a large polysaccharide is not easily absorbed through the gastro-intestinal system, preventing the fucoidan from being made systemically in effective amounts available when orally ingested. The poor uptake of fucoidan when orally ingested is reviewed by Fitton J. et al, Therapies from Fucoidan: An Update, Mar. Drugs 2015, 13, 5920-5946 and in the subsequent update Fiton J et al, Therapies from Fucoidan: An Update, Mar. Drugs 2015, 13, 5920-5946.

Extracts of fucoidan are commercially available and hence concentrated and elevated doses of fucoidan can be administered in an effort to increase uptake, but due the poor absorption of fucoidans this may still not lead to increased uptake and is an inefficient use of product.

To address the problem of poor uptake after oral administration, alternative modes of administration can be pursued. For example, fucoidan may be administered via the parenteral route, i.e. intravenous. This requires the availability of trained staff and that patients or target groups provide expanded levels of compliance for this type of administration, hence oral administration is preferable. Thus, using the parenteral fucoidan administration does not offer a satisfactory solution when it comes to addressing the issue of insufficient fucoidan uptake.

Hence, it is an objective of the invention to provide a composition comprising fucoidan for oral administration which provides an effective amount of fucoidan to the user. Another object of the invention is to efficiently provide the benefits of fucoidan in terms of material use. In particular, an object of the invention is to provide a composition providing improved uptake of fucoidan.

### Summary of the invention

In the light of the above, it is an object of the invention to provide a composition, and therapeutic uses thereof, which may be advantageous in terms of fucoidan uptake and enhanced bioactivity.

These and other objects of the invention are achieved through a first aspect of the invention, where there is provided a composition for oral administration, comprising fucoidan from a first source of fucoidan and from a second source of fucoidan, the first source of fucoidan being dried seaweed and the second source of fucoidan being a fucoidan extract from seaweed, wherein a weight ratio of the second source to the first source of fucoidan is in the range of 10 % to 50 % and further wherein the first source and second source constitutes at least 60 % by weight of the composition.

The second source of fucoidan is a fucoidan extract from a brown seaweed, brown seaweeds being the species which contain fucoidan. The extract is provided to achieve a desired amount of fucoidan in the composition. Using an extract also allows the composition to be suitably concentrated with respect to fucoidan, which enables practical administration of the desired amount. If the composition was too dilute, it would not be useful as the amount of composition to be administered would be excessive. For example, if the composition is provided in the form of capsules, a dilute composition would entail a large number of capsules to be consumed daily, possibly spaced throughout a day, which is not desirable for the user and increases the risk of non-adherence to a specified dosage regimen. In another example, where the composition is added to other foodstuffs, such as a beverage, large amounts of the composition could affect the palatability or other properties of the foodstuff, which also increasing the risk of non-adherence to the specified dosage regimen.

The first source of fucoidan in the composition is provided as a dried seaweed. As the dried seaweed is a source of fucoidan, it is understood that it is a dried brown seaweed. The dried seaweed could be in the form of a granulate. The dried seaweed is a source of a variety of dietary fibre which may be at least partially indigestible by the user, but which stimulates the microbiota of the user, which may lead to an improved gut health and/or microbiota variety and population. When a subject has a healthier or more diverse gut microbiota, it is contemplated that the uptake of fucoidan by the subject is improved, assisted by the microorganisms in the gut. The microorganisms in the gut may digest fucoidan, leaving smaller units of the fucoidan molecule which may be more easily absorbed through the intestine of the user, than larger fucoidan molecules. Without being bound by theory, it is contemplated that by giving a subject combination of fucoidan extract and dried seaweed containing fucoidan, may attune the microbiota of the subject to fucoidan digestion, which may lead to increased uptake and efficient utilisation of the fucoidan from the extract. This may be especially advantageous for subjects whose diet does not contain brown seaweeds, such as in Europe or North America, and whose microbiota may thus not naturally include species or enzymes capable of digesting fucoidan. Furthermore, the dried seaweed may include microorganisms from the environment in which the seaweed was grown and from which it was harvested, which microorganisms may have genes for enzymes capable of digesting fucoidan, and these genes may then be incorporated into the microbiota of the user.

In addition, stimulation and improving gut health by ingesting dried seaweed is a benefit to the user in its own right, as gut health impacts the general health of a subject, for example in maintaining immune homeostasis and inflammation responses, as is the subject of increasing interest in the medical field.

The second source of fucoidan, the extract, is high in concentration, e.g. at least 80 % purity with respect to fucoidan, and the first source, the dried seaweed, will typically be present in excess of the extract in terms of weight, although a 1:1 ratio of extract to dried seaweed is also contemplated. Hence, in the most general embodiment of the composition the weight ratio of the second source, extract, to the first source, dried seaweed, is in the range of 10 % to 50 %. The weight ratio being the fraction of extract (numerator) and dried seaweed (denominator) expressed as percentage.

To achieve the beneficial effects of the first and second sources, the composition is in a form which can readily be used by user, with which adherence to the specified dosage is promoted, and which can contain sufficient amount fucoidan. Hence, in the most general embodiments the first and second source of the composition constitutes at least 60 % by weight of the composition. In this way, the composition can be provided as capsules where a few capsules, e.g. 1, 2 or 3, can provide a daily dose of the composition or the daily dose can be incorporated into other foodstuff without affecting the properties of the foodstuff.

Preferably the first source and second source constitute, at least 65 %, at least 70 %, at least 75 %, at least 80 %, least 85 %, at least 90 % or 95 % of the composition by weight. The first and second source may also constitute the entirety of the composition. The remaining constituents of the composition can be further active ingredients which will be described in greater detail below, or other components such as stabilisers. If the composition is provided as a capsule, i.e. encapsulated in single-piece or two-piece capsules, the capsule material is not counted when calculating the percentage constituted by the first and second source. That is, the capsule material is disregarded from the total weight in that calculation.

In some embodiments, the weight ratio of the second source to the first source is in the range of 15 % to 45 %, 20 % to 40 %, 25 % to 35 %, or 30 % to 35 %.

These ratios may provide a composition which has both an effective amount of fucoidan and sufficient content of dried seaweed to stimulate the gut microbiota.

In a preferred embodiment, the weight ratio of the second source to the first source is in the range of 25 % to 35 %, and the first source and second source constitutes at least 80 % of the composition. In a further development, the first source and second source constitute 100 % of the composition.

The fucoidan extract in the context of the invention is fucoidan which has been isolated and concentrated from a brown seaweed. Fucoidan extracts are available commercially and a number of extraction methods are known in the art. For example, Qingdao Bright Moon Seaweed Group offers suitable extracts having greater than 90 % fucoidan purity. Sigma Aldrich also offers fucoidan extract at greater than 95 % purity from *Fucus Vesiculosus* (CAS 9072-19-9).

In some embodiments, the fucoidan content of the second source, the extract, is at least 80 % by weight, preferably at least 85 % by weight and more preferably at least 90 % by weight.

In some embodiments, the first source of fucoidan is dried seaweed selected from *Fucus,* preferably *Fucus vesiculosus.*

In some embodiments, the second source of fucoidan is an extract from a seaweed selected from *Laminaria,* preferably *Laminaria japonica.*

Fucoidan is a group of polysaccharides where the structure can vary across different seaweed species. They may have different contents of fucose, different glycosidic linkages, sulphate ester positions, sulphation degrees and different molecular weight. The variance of the structure may affect the biochemical activities of the fucoidan. It is presently preferred that the second source, i.e. the dried seaweed, used in the composition is from the genus *Fucus* and more preferably *Fucus vesiculosus* also known as bladderwrack. The first source of fucoidan, i.e. the extract, is preferably from another brown algae species, preferably from the genus *Laminaria* or *Saccharina* and more preferably *Laminaria japonica,* which is sometimes known under the name *Saccharina japonica.*

In a preferred embodiment, the weight ratio of the second source to the first source is in the range of 25 % to 35 %, and the first source and second source constitutes at least 80 % of the composition, and the first source of fucoidan is dried *Fucus vesiculosus* and the second source of fucoidan is a fucoidan extract from *Laminaria japonica.* In a further development, the first source and second source constitute 100 % of the composition.

The composition may comprise further ingredients. In some embodiments the composition further comprises an edible thickening agent, preferably agar. Thickening agents promotes satiety in the user, which may of special benefit for certain users as an aid in managing a healthy diet, as satiety reduces the tendency of non-adherence to a dietary plan. Other thickening agents may be used, such as alginin or carrageenan.

The dried seaweed will typically be in the form of a granulate, which can be made by washing the fresh seaweed, drying it and milling/grinding it. The drying step can be performed at moderate temperatures, e.g. in the range of 40 to 80 degrees Celsius, so as to reduce damage the constituents of the seaweed and/or any microorganisms present on the seaweed. In some embodiments, the dried seaweed may be freeze dried, which may advantageously preserve constituents of the seaweed and/or any microorganisms present on the seaweed.

The composition may be provided in a variety of oral dosage forms, but preferably it is provided as capsules containing the composition. Capsules provide a practical way of controlling the dosage, and as the first and second source of fucoidan constitute the majority of the composition, and even the entirety of the composition in some embodiments, it is possible to achieve the desired dosage in a few capsules, reducing the risk of mistakes in dosing and the user not complying with a dosage regimen due to an excessive capsule number.

The composition as described above may be used as a medicament. Fucoidan has anti-viral, anti-inflammatory and anti-cancer activity and may serve as immunomodulant, stimulating the immunity of the user. The composition can provide these effects along with promotion of gut health provided by the dried seaweed. The combination of fucoidan extract and dried whole seaweed, may serve to improve uptake of the fucoidan and provide an effective amount of fucoidan systemically to the user. Improved uptake also allows for more efficient use of fucoidan, as a smaller dosage can provide an effective amount.

The composition may be used specifically as an immunomodulating medicament, promoting immunity. This may be observed by increased production/proliferation of serum antibodies, cytokines or immune cells.

The composition may be used in the treatment of cancer.

The composition may be used in the treatment of bacterial or viral infections.

When the composition is used as a medicament or in methods of treatment, the composition is administered orally in a total daily amount containing 1000 to 2000 mg fucoidan, preferably 1400 to 1600 mg, more preferably about 1500 mg.

As previously described the fucoidan content of the extract is high, i.e. above 80 % or 90 %, and the extract typically provides the majority of the fucoidan in the composition. The first source fucoidan, i.e. the dried seaweed, is dried whole seaweed and the fucoidan content can vary depending on season, harvest area, species and whether specific parts of the seaweed is used, for example reproductive tissue in the seaweed is reported to have higher content of fucoidan. Typically, the amount of fucoidan in the dried seaweed is in the range of 5 to 20 % by weight. This contribution is also counted in the dosage. It is presently preferred that the composition is consumed in amount which contains 1000 to 2000 mg fucoidan daily; preferably 1200 to 1800 mg or 1250 to 1750 mg; more preferably 1300 to 1700 mg, 1350 to 1650 mg, 1400 to 1700 or 1400 to 1600 mg; most preferably about 1500 mg. The present inventors believe these dosages will provide an effective amount of fucoidan to the user, stimulate the gut microbiota of the user and improve fucoidan uptake.

The dosages may also be expressed in terms of the daily amount of the composition to be ingested. Hence, the composition may be administered orally in a total daily amount of 2.5 to 7 g, preferably 3 to 6 g, more preferably 4 to 5 g, most preferably about 4 g.

In a preferred embodiment of the composition, the weight ratio of the second source to the first source is in the range of 25 % to 35 %, and the first source and second source constitutes at least 80 % of the composition, and the first source of fucoidan is dried *Fucus vesiculosus* and the second source of fucoidan is a fucoidan extract from *Laminaria japonica.* This preferred embodiment may be administered orally in a total daily amount of 3 to 6 g, preferably 3 to 5 g, more preferably about 4 g.

Another specific embodiment of the composition consisting of the first and second source, at a weight ratio between the second and first source of about 33 %, having a fucoidan content in the extract of 90 weight% or more and a fucoidan content of the dried seaweed of about 15 weight%, can be given in a daily amount of about 4 to 4.5 grams providing a daily dose of about 1.5 g fucoidan.

An alternative embodiment of the composition consisting of the first and second source and also agar, at a weight ratio between the second and first source of about 33 %, having a fucoidan content in the extract of about 90 weight% or more and a fucoidan content of the dried seaweed of about 15 weight%, agar being 15 weight% of the total weight of the composition, can be given in a daily amount of about 4.8 to 5.2 grams providing a daily dose of about 1.5 g fucoidan.

The daily amount of composition to be consumed is adjusted according to the weight ratio of the first and second source, the respective contents of fucoidan in the extract and in the dried seaweed, and the fraction of the composition constituted by the extract and the dried seaweed, to obtain the desired fucoidan dose as previously described. It is understood that at a high weight ratio of extract to dried seaweed and/or at a high extract fucoidan content, the daily amount of composition to be consumed will be at the lower end of the above ranges, whereas low weight ratios will increase the daily amount to be consumed.

For example, 7 g of a composition where the first and second source constitute 80 weight% of the composition, the weight ratio of second to first source being 10 %, provides a daily dose of about 1.2 g fucoidan, when the fucoidan content of the extract is 90 weight% and the fucoidan content of the dried seaweed is 15 weight %.

In another example, 2.5 g of a composition where the first and second source constitute 100 weight% of the composition, the weight ratio of second to first source being 50 %, provides a daily dose of about 1.6 g fucoidan, when the fucoidan content of the extract is 90 weight% and the fucoidan content of the dried seaweed is 15 weight %.

The dosages described above refer to administration to human users. The dosages described above are suitable when using the composition as a medicament, an immunomodulatory medicament, in the treatment of cancer or in the treatment of viral or bacterial infections.

The first and second source of fucoidan may be the only sources of fucoidan in the composition.

In some embodiments, the composition consists of the first and second source of fucoidan.

In a further aspect of the invention, there is provided a supplement which comprises the composition for oral administration as described herein and collagen.

Collagen is a main component in connective tissue, such as skin, and ingesting collagen may improve the skin-health of the user. Native collagen production is reduced with age and the composition may serve to supplement the native collagen production. Improvement in skin-health may be seen as increased skin elasticity, increased skin smoothness, fewer or smaller wrinkles, and/or increased skin moisture. Fucoidan have also been associated with skin-health improvement. Cf. for example Fiton et al, "Topical Benefits of Two Fucoidan-Rich Extracts from Marine Macroalgae", Cosmetics 2015, 2, 66-81, showing *in vitro* that fucoidan serves as an inhibitor of collagenase which is key in skin-ageing, and in a clinical test an improvement in skin brightness and wrinkle appearance i.a.

It is contemplated by the inventor, that by combining the composition of the invention, which may provide improved fucoidan uptake, and collagen, in the supplement, may lead to even further improvements in skin-health.

The collagen is preferably of porcine origin, i.e. obtained from the skin and/or bones of pigs, but may also be obtained from e.g. the skin of fish. The collagen may be hydrolysed.

The composition used in the supplement may be any of the embodiments described above.

For example, in one embodiment of the supplement, the composition is the previously described preferred embodiment, in which the weight ratio of the second source to the first source is in the range of 25 % to 35 %, and the first source and second source constitutes at least 80 % of the composition, and the first source of fucoidan is dried *Fucus vesiculosus* and the second source of fucoidan is a fucoidan extract from *Laminaria japonica.*

In some embodiments, the relative amounts of composition and collagen in the supplement is chosen according to the desired daily dosages of each. As previously described, it is presently preferred that the composition is consumed in amount which contains 1000 to 2000 mg fucoidan; preferably 1200 to 1800 mg or 1250 to 1750 mg; more preferably 1300 to 1700 mg, 1350 to 1650 mg, 1400 to 1700 or 1400 to 1600 mg; most preferably about 1500 mg. The presently preferred amount of collagen for daily consumption is in the range of 2000 to 6000 mg, more preferably 2500 to 5000 mg.

Hence, a preferred embodiment of the supplement comprises relative amounts of composition and collagen, such that when 1500 mg is ingested, 2500 to 5000 mg of collagen is ingested.

Typically, collagen constitutes 30 to 70 % by weight of the supplement, preferably 40 to 60 % by weight.

In some embodiments of the supplement, the supplement consists of collagen and the composition.

### Detailed description

Table I below provides an example of an embodiment of the composition according to the invention, wherein the first and second source are the only components of the composition.

**Table I**

| **Component** | **Value** |
|---|---|
| Fucoidan extract of *Laminaria Japonica,* >90% purity | 1 g |
| Dried granulate of *Fucus vesiculosus* | 3 g |
| Total mass | 4 g |
| Weight ratio of extract to dried seaweed | 33 weight % |
| Dosage, daily | 4 to 5 g |

The dried seaweed in the composition of Table I has a fucoidan content of about 15 weight% and extract purity of at least 90 weight%. Hence, a dosage of 5 g, assuming 90 weight% extract purity provides a fucoidan dosage of 1.7 g, whereas 4 g assuming 100 % extract purity provides 1.5 g.

Another embodiment of the composition is shown in Table II which includes agar as an edible thickening agent.

**Table II**

| **Component** | **Value** |
|---|---|
| Fucoidan extract of *Laminaria Japonica,* >90% purity | 1.05 g |
| Dried granulate of *Fucus vesiculosus* | 3.20 g |
| Agar | 0.75 g |
| Total mass | 5 g |
| Weight ratio of extract to dried seaweed | 33 weight % |
| Extract+Dried seaweed weight fraction | 85 weight % |
| Dosage, daily | 5 to 6 g |

The dried seaweed in the composition of Table I has a fucoidan content of about 15 weight% and extract purity of at least 90 weight%. Hence, a dosage of 6 g, assuming 90 weight% extract purity provides a fucoidan dosage of 1.7 g, whereas 5 g assuming 100 % extract purity provides 1.5 g. The agar will make the user feed satiated which may help the user to adhere to a healthy diet, preventing the urge to eat outside regular meals.

The composition as disclosed herein may be especially suitably for users engaging in regular and possibly strenuous physical activity, such as athletes. Physical activity especially high intensity physical activity may in some cases lead to a weakened immune system and the weakened or impaired immunity may be short term, i.e. from a few hours to a few days after physical exertion, Walsh NP, Gleeson M, Pyne DB, et al. Position statement part two: maintaining immune health. Exerc Immunol Rev. 2011;17:64-103, see especially pages 67 and 70. The impairment of immune function is most pronounced when the exercise is continuous, prolonged (>1.5 h), of moderate to high intensity (55-75% maximal O2 uptake), cf. Walsh NP et al.

Hence, the composition as disclosed herein may be especially suitable for a group of subjects or patients engaging in sessions of high-intensity physical activity, each such session including at least 1 hour at 50 % of the maximal oxygen-uptake of the user. The group of subjects or patients may be further characterized by engaging such sessions at least 1-7, 2-6, 3-5 or 3 times per week as measured across a 30-day period.

The composition as disclosed herein may contribute to maintaining normal immune function and/or reduce the detrimental effect of high intensity physical activity on the immune system. The fucoidan itself can act as immunomodulant and the composition also promotes a healthy gut microbiota which can also affect the immune system of the user.

It is presently contemplated that the increased uptake of fucoidan by the user, when ingesting the composition as disclosed herein, compared to pure fucoidan, may be evaluated by analysing the fucoidan content of serum or urine of a test subject. An ELISA method for detecting fucoidan in such samples is described by Yoshiharu Tokita et al., Development of a fucoidanspecific antibody and measurement of fucoidan in serum and urine by sandwich ELISA, Biosci Biotechnol Biochem 2010;74(2):350-7.

The fucoidan content of an extract, or of the composition in general, can be measured by the method disclosed in standard SC/T 3404-2012. An alternative measurement method is disclosed in CN114047284A. Another method is described in United States Food and Drug Administration GRAS Notice, GRN No. 661, "Fucoidan concentrate from Fucus vesiculosus", filed 19 August 2016, by Marinova Pty. Ltd ,cf. Appendix II: CO5, "Determination of Fucoidan Content".

As previously described fucoidan extracts from brown seaweeds can be obtained commercially. Fucoidan extracts from Qingdao Bright Moon Seaweed Group are suitable for the compositions as disclosed herein.

A number of fucoidan extraction methods are known in the art, for example in CN109134680A. The typical methods a hot-water or hot-acid extraction followed by alcohol precipitation of fucoidan, and a review of extraction methods is provided by Ale MT, Mikkelsen JD, Meyer AS, Important determinants for fucoidan bioactivity: a critical review of structure-function relations and extraction methods for fucose-containing sulfated polysaccharides from brown seaweeds, Mar Drugs. 2011;9(10):2106-2130.

Tables III and IV show embodiments of the supplement according to the invention.

**Table III**

| **Component** | **Value** |
|---|---|
| Fucoidan extract of *Laminaria Japonica,* >90% purity | 1 g |
| Dried granulate of *Fucus vesiculosus* | 3 g |
| Collagen (Porcine) | 2.5 g |
| Total mass | 6.5 g |
| Weight ratio of extract to dried seaweed | 33 weight % |
| Dosage, daily | 6.5 to 7.5 g |

The dried seaweed in the composition of Table III has a fucoidan content of about 15 weight% and extract purity of at least 90 weight%. Hence, a dosage of 7.5 g of supplement, assuming 90 weight% extract purity provides a fucoidan dosage of about 1.5 g and 2.9 g collagen, whereas 6.5 g assuming 100 % extract purity provides about 1.5 g fucoidan and 2.5 g collagen.

**Table IV**

| **Component** | **Value** |
|---|---|
| Fucoidan extract of *Laminaria Japonica,* >90% purity | 1 g |
| Dried granulate of *Fucus vesiculosus* | 3 g |
| Collagen (Porcine) | 5 |
| Total mass | 9 g |
| Weight ratio of extract to dried seaweed | 33 weight % |
| Dosage, daily | 9 to 10 g |

The dried seaweed in the composition of Table IV has a fucoidan content of about 15 weight% and extract purity of at least 90 weight%. Hence, a dosage of 10 g of supplement, assuming 90 weight% extract purity provides a fucoidan dosage of about 1.5 g and 5.8 g collagen, whereas 9 g assuming 100 % extract purity provides about 1.5 g fucoidan and 5 g collagen.

A composition for oral administration as used herein is a product which is to be ingested.

The first source of fucoidan is a dried seaweed, the seaweed being fucoidan-containing species, i.e. a brown seaweed/macroalgae.

The second source of fucoidan is an extract of fucoidan from seaweed, i.e. fucoidan which has been isolated and concentrated from seaweed.

The weight ratio of the second to first source of fucoidan is the mass fraction of the mass of second source (extract) to the mass of the first source (dried seaweed) in the composition, the second source being the numerator of the fraction.

Fucoidan as used herein refers to a group of sulphated polysaccharide with a backbone of mainly fucose found in the cell walls of many brown macroalgae (brown seaweeds). The fucose-backbone is α-L-fucopyranose units, which can be linked by (1→3) and (1→4) glycosidic bonds, sometimes with repeated (1→3)-links or sometimes with alternating (1→3)- and (1→4)-links. The sulphate groups is generally found on the C2 and/or C4 position. Other monosaccharides may be present such as galactose, mannose, xylose and glucose. The structure is diverse and may vary according to the brown seaweed species. A review of the current structural understanding may be found in Ale MT, Mikkelsen JD, Meyer AS, Important determinants for fucoidan bioactivity: a critical review of structure-function relations and extraction methods for fucose-containing sulfated polysaccharides from brown seaweeds, Mar Drugs. 2011;9(10):2106-2130.

## Claims

1. A composition for oral administration, comprising fucoidan from a first source of fucoidan and from a second source of fucoidan,
the first source of fucoidan being dried seaweed, and
the second source of fucoidan being a fucoidan extract from seaweed,
wherein a weight ratio of the second source to the first source of fucoidan is in the range of 10 % to 50 % and further wherein the first source and second source constitutes at least 60 % by weight of the composition.

2. The composition according to claim 1, wherein the weight ratio of the second source to the first source is in the range of 15 % to 45 %, 20 % to 40 %, 25 % to 35 %, or 30 % to 35 %.

3. The composition according to claim 1 or 2, wherein the first source and second source constitutes at least 65 %, at least 70 %, at least 75 %, at least 80 %, least 85 %, at least 90 % or 95 % of the composition by weight.

4. The composition according to claim 1,
wherein the weight ratio of the second source to the first source is in the range of 25 % to 35 %, and the first source and second source constitutes at least 80 % of the composition.

5. The composition according to any one of the preceding claims,
wherein the fucoidan content of the second source is at least 80 % by weight, preferably at least 85 % by weight and more preferably at least 90 % by weight.

6. The composition according to any one of the preceding claims, wherein the first source of fucoidan is dried seaweed selected from *Fucus,* preferably *Fucus vesiculosus.*

7. The composition according to any one of the preceding claims, wherein the second source of fucoidan is an fucoidan extract from a seaweed selected from *Laminaria* or *Saccharina,* preferably *Laminaria japonica.*

8. The composition according to claim 4,
wherein the first source of fucoidan is dried *Fucus vesiculosus* and the second source of fucoidan is a fucoidan extract from *Laminaria japonica.*

9. The composition for oral administration according to any one of the preceding claims further comprising an edible thickening agent, preferably agar.

10. The composition according to any one of the preceding claims, wherein the dried seaweed is a granulate.

11. The composition according to any of the preceding claims, said composition being an oral dosage form, such as a capsule.

12. The composition according to any one of claims 1 to 11, for use as a medicament.

13. The composition for oral administration according to any one of claims 1 to 11, for use as an immunomodulating medicament or for use in treatment of cancer or a viral or bacterial infection in a subject in need thereof.

14. The composition for use according to claim 12 or for use according to claim 13, wherein the composition is administered orally in a total daily amount containing 1000 to 2000 mg fucoidan, preferably 1250 to 1750 mg, more preferably 1350 to 1650 mg, most preferably about 1500 mg.

15. The composition for use according to claim 12 or for use according to claim 13,
wherein the composition is administered orally in a total daily amount of 2.5 to 7 g, preferably 3 to 6 g, more preferably 4 to 5 g, most preferably about 4 g.

16. A supplement, comprising
collagen, and
a composition comprising fucoidan from a first source of fucoidan and from a second source of fucoidan,
the first source of fucoidan being dried seaweed, and
the second source of fucoidan being a fucoidan extract from seaweed,
wherein a weight ratio of the second source to the first source of fucoidan is in the range of 10 % to 50 % and further wherein the first source and second source constitutes at least 60 % by weight of the composition.

17. The supplement according to claim 16, wherein collagen constitutes 30 to 70 % by weight of the supplement, preferably 40 to 60 % by weight.
